# FASCICULE DE BREVET EUROPEEN

(11) **EP 1 461 601 B1**
(45) Date de publication et mention de la délivrance du brevet: **23.08.2017**
(21) Numéro de dépôt: 02799106.6
(22) Date de dépôt: 20.12.2002
(51) Int. Cl.: G01N 21/64, A61B 1/04, G02B 23/26, A61B 5/00

(54) **APPAREILLAGE DE SPECTROSCOPIE D'AUTOFLUORESCENCE SUBSURFACIQUE**
VORRICHTUNG ZUR AUTOFLUORESZENZSPEKTROSKOPIE AUS DEM INNERN
EQUIPMENT FOR SPECTROSCOPY OF SUBSURFACE AUTOFLUORESCENCE

(30) Priorité: 28.12.2001 FR 0116981
(43) Date de publication de la demande: 29.09.2004
(73) Titulaire: Mauna Kea Technologies, 75010 Paris (FR)
(72) Inventeur: GENET, Magalie, F-78280 Guyancourt (FR); BOURG-HECKLY, Geneviève, F-75003 Paris (FR); VILETTE, Sandrine, F-75013 Paris (FR); LACOMBE, François, F-92370 Chaville (FR); LOISEAU, Alexandre, F-75007 Paris (FR); ABRAT, Benjamin, F-75008 Paris (FR)
(74) Mandataire: Pontet Allano & Associes
(86) Numéro de dépôt international: PCT/FR2002/004480
(87) Numéro de publication internationale: WO 2003/060493

(56) Documents cités:
- EP-A- 1 157 655
- WO-A-00/16151
- WO-A-99/47041

## Description

La présente invention concerne un appareillage de spectroscopie d'autofluorescence subsurfacique. Plus particulièrement, l'appareillage selon l'invention est du type utilisant un signal d'excitation véhiculé par une ou plusieurs fibres optiques souples.

Les domaines d'application de l'invention sont l'analyse spectroscopique de tissus biologiques in vivo sur l'homme ou l'animal, externes ou internes et accessibles à l'aide d'un canal opérateur d'endoscope dans lequel on peut introduire les fibres optiques, et également l'analyse ex-vivo d'échantillons tissulaires provenant de prélèvements biopsiques, et l'analyse in-vitro de cultures en biologie cellulaire.

Actuellement sont visés les domaines médicaux de la gastro-entérologie, la pneumologie, la gynécologie, l'urologie, l'ORL, la dermatologie, l'ophtalmologie, la cardiologie et de la neurologie.

Les tissus biologiques contiennent des fluorophores endogènes susceptibles, en réponse à une excitation lumineuse de longueur d'onde appropriée, d'émettre une fluorescence dans un domaine spectral allant de l'UV proche au visible, appelée autofluorescence. Cette dernière résulte du recouvrement d'émissions de différents fluorophores, et dépend du métabolisme cellulaire, de la structure et de la vascularisation des tissus, qui varient suivant la nature saine ou tumorale des tissus. Il en résulte que la fluorescence des tissus sains et tumoraux présentent de fortes différences tant sur le plan de l'intensité émise que sur la forme du spectre. L'analyse du spectre d'autofluorescence fournit des indicateurs permettant une "biopsie optique".

Dans les appareillages existants, l'illumination du site que l'on souhaite analyser de manière spectroscopique se fait couramment avec un faisceau d'excitation de fibres optiques qui est divergent, excitant un volume, et la réception du signal de fluorescence s'effectue au moyen de fibres de détection adjacentes notamment périphériques. Cela entraîne une mauvaise résolution, avec un mélange d'informations et une augmentation du nombre de faux positifs.

La présente invention a pour but de pallier ces inconvénients.

Dans la demande de brevet WO 99/47041 on propose un appareillage pour l'imagerie subsurfacique spectrale et confocale d'un tissu biologique. Il a été par ailleurs proposé des techniques d'imagerie confocale à haute résolution spatiale destinées à observer un plan de coupe XY à différentes profondeurs du site observé, notamment exposée dans la demande de brevet WO OO/16151.

Ces techniques mettent en oeuvre un faisceau ordonné de fibres optiques souples (notamment plusieurs dizaines de milliers) avec, côté observateur, une source lumineuse et un système d'injection de fibres permettant d'illuminer une seule fibre et, côté site observé, une tête optique permettant de focaliser le faisceau sortant de la fibre illuminée en un point situé dans un plan de coupe XY, à une profondeur donnée du site observé. Un système de balayage de fibres permet de balayer les fibres une à une à très grande vitesse. Chaque fibre est utilisée tour à tour pour véhiculer le faisceau d'illumination et également le faisceau de retour correspondant provenant du site observé. L'obtention d'une haute résolution spatiale est due au fait que l'on focalise le faisceau en un point et également au caractère confocal résidant dans le filtrage spatial du site observé par les mêmes fibres que celles ayant servi à l'illumination. Cela permet de réceptionner exclusivement le signal provenant du site observé et de réaliser une image point par point.

La présente invention a pour but de proposer un appareillage qui permette une analyse spectroscopique également à haute résolution spatiale et à une profondeur donnée du site observé.

Elle propose un appareillage d'analyse spectroscopique d'autofluorescence d'un tissu selon l'objet de la revendication 1. La présente invention est ainsi basée sur certains des moyens cités plus haut pour réaliser une image confocale, à savoir véhiculer sur la ou les mêmes fibres le signal d'excitation et en retour le signal émis, et la mise en oeuvre d'une tête optique focalisant le signal d'excitation en un point en profondeur. La focalisation combinée au caractère confocal (obtenu grâce au retour du signal d'autofluorescence par la ou les mêmes fibres optiques), permet d'obtenir une haute résolution spatiale. L'avantage par rapport à une spectroscopie grand champ est que l'on peut réaliser une sélection spatiale très précise de la zone d'analyse et que l'on réduit ainsi beaucoup les risques d'erreurs et de faux positifs.

Les moyens optiques de la tête optique comprennent un système de lentilles formant un objectif de focalisation adapté à transcrire la répartition spatiale de la tache focale en sortie du faisceau de fibres et la qualité du front d'onde et à minimiser la réflexion parasite s'opérant en sortie du faisceau de fibres.

Selon la présente invention, le nombre de fibres optiques du faisceau comprend une pluralité de fibres (notamment plusieurs dizaines de milliers) pouvant être toutes excitées ou par sous-ensembles sélectionnés selon les dimensions de la zone d'excitation que l'on recherche.

La zone d'excitation selon l'invention se situe dans un plan XY perpendiculaire à l'axe optique que l'on peut régler à différentes profondeurs, allant sensiblement de 50 à 400 *µ*m. Sa dimension dépend du diamètre de l'ensemble de fibres utilisé (appelé ci-après le diamètre utile du faisceau de fibres), et des caractéristiques optiques de focalisation de la tête optique. Lorsque l'on utilise un faisceau constitué d'une multitude de fibres optiques souples, l'appareillage peut avantageusement comprendre des moyens permettant de régler le diamètre du faisceau d'excitation émis par la source pour qu'il excite en continu soit l'ensemble des fibres soit un sous-groupe de fibres permettant d'obtenir une taille appropriée pour la zone d'excitation. Ces moyens sont par exemple constitués d'une lentille d'adaptation de faisceau ou d'un système afocal de grandissement approprié.

La présente invention propose également un appareillage comportant en outre des moyens permettant d'obtenir conjointement une image confocale du site d'analyse. Une telle possibilité de couplage permet d'augmenter avantageusement le degré de certitude d'un diagnostic. Grâce à l'invention, on pourra obtenir simultanément et en temps réel, sur un point focalisé en profondeur, des informations :
- de type histologique par imagerie confocale ; et
- de type spectroscopique renseignant sur la nature du site observé.

Elle propose un appareillage tel que défini ci-dessus, le faisceau de fibres comportant une pluralité de fibres optiques, caractérisé en ce qu'il comporte en outre des moyens pour réaliser conjointement une image confocale de la zone d'analyse, comprenant :
- une source d'illumination,
- un détecteur du signal de retour pour analyse,
- un moyen de séparation du signal d'illumination et dudit signal de retour,
- des moyens pour coupler le signal d'excitation pour l'analyse spectroscopique et le signal d'illumination pour l'imagerie confocale, avant l'entrée dans le moyen d'injection dans le faisceau de fibres optiques,
- un moyen de balayage rapide des fibres une à une situé en amont du moyen d'injection dans le faisceau de fibres, et
- un système de filtrage spatial à l'entrée du détecteur de signal adapté à sélectionner le signal de retour provenant de la fibre illuminée,
le moyen d'injection dans le faisceau de fibres présentant une répartition spatiale de l'intensité de la tache focale égale au diamètre de coeur d'une fibre, chaque fibre étant illuminée tour à tour et de manière adressée.

Selon l'invention, la voie tomographique et la voie spectroscopique utilisent avantageusement en commun le moyen d'injection dans le faisceau de fibres, le faisceau de fibres lui-même et la tête optique de focalisation. Pour l'acquisition d'une image, les fibres sont illuminées tour à tour et une à une. Pour l'acquisition d'un spectre d'autofluorescence, les fibres peuvent être excitées toutes ou par sous-groupes, en fonction des dimensions de la zone d'analyse.

L'utilisation d'un faisceau de fibres optiques souples peut être avantageuse pour un système de tests automatiques dans lequel avantageusement le faisceau de fibres, avec à son extrémité la tête optique, est manipulé de manière automatisée comme un bras de mesure sur une matrice d'échantillons.

La présente invention sera mieux comprise et d'autres avantages apparaîtront à la lumière de la description qui va suivre de deux exemples de réalisation, description faite en référence aux dessins sur lesquels :
- la figure 1 illustre schématiquement un premier exemple de réalisation d'appareillage de spectroscopie selon l'invention ;
- la figure 2 illustre schématiquement un deuxième exemple de réalisation d'appareillage comprenant un couplage analyse spectroscopique et imagerie confocale ; et
- la figure 3 illustre schématiquement une variante de réalisation de l'appareillage de la figure 2.

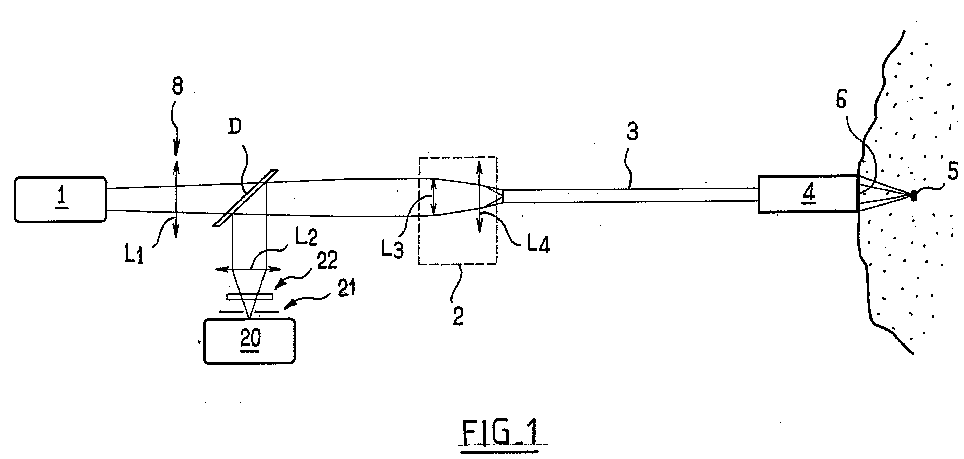

Selon l'exemple de réalisation choisi et représenté sur la figure 1, il est proposé un appareillage pour réaliser une analyse spectroscopique subsurfacique à une profondeur donnée, comportant une source 1 produisant un signal d'excitation, un moyen d'injection 2 dudit signal dans un faisceau 3 ordonné de fibres optiques au bout duquel est ménagé une tête optique 4 adaptée à modifier le signal d'excitation sortant dudit faisceau de fibres optiques 3 pour créer un faisceau convergent focalisé sur une zone 5 sous-jacente de la zone de contact 6 avec la tête optique 4.

La source 1 utilisée est choisie pour permettre une excitation des fluorophores endogènes présents dans les tissus biologiques du site observé, notamment dans une plage de longueur d'onde de 300-500 nm. Typiquement on peut utiliser une diode laser à 405+/- 10 nm. D'autres sources telles que les lasers solides ou lasers à gaz avec ou non générateurs d'harmoniques peuvent également convenir avec d'autres longueurs d'ondes afin d'exciter d'autres fluorophores endogènes.

L'appareillage comporte un moyen d'adaptation 8 de faisceau émis par la source 1 comprenant ici une lentille L1 qui est correctement couplée au moyen d'injection 2 dans le faisceau 3 de fibres optiques. La combinaison optique permet d'adapter la taille du faisceau laser au diamètre utile dudit faisceau de fibres optiques 3, correspondant à l'ensemble ou à un sous-ensemble de fibres effectivement utilisées. Elle permet en outre ici d'agrandir le diamètre de la tache de focalisation à l'entrée du faisceau 3 de fibres et par conséquent d'agrandir la taille de la tache formée sur le tissu. Cela permet de réduire l'irradiance (c'est à dire la quantité de puissance par unité de surface) sur le tissu, et ainsi de respecter les normes d'éclairement des tissus biologiques.

L'appareillage comporte également un moyen adapté à séparer deux longueurs d'onde, la longueur d'onde d'excitation et le signal d'autofluorescence émis. Une lame dichroïque D est utilisée ici à cet effet réalisant une transmission maximale à la longueur d'onde d'illumination et une réflexion maximale dans le domaine spectral de fluorescence.

Le signal à la longueur d'onde d'excitation est dirigé en sortie de la lame D vers le moyen optique d'injection 2 dans le faisceau de fibres 3. Ce moyen doit présenter le minimum d'aberrations et ne doit pas dégrader la qualité du front d'onde afin de réaliser une tache de focalisation proche de la limite de diffraction pour ainsi réaliser un couplage optimal avec le faisceau de fibres 3. Le moyen choisi ici est constitué d'un doublet sur mesure L3 et d'un triplet standard L4. Le doublet L3 permet de corriger les aberrations résiduelles du triplet L4, à savoir la courbure de champ. Toute autre combinaison optique présentant une qualité de front d'onde WFE ("Wave Front Error") de l'ordre de λ/8 et une répartition spatiale de l'intensité de la tache focale PSF ("Point Spread Function") égale au diamètre utile du faisceau de fibres 3 peut convenir.

Le faisceau de fibres 3 permet d'accéder à la zone d'analyse en déportant la source 1. Pour une application endoscopique, il doit présenter un diamètre et un rayon de courbure permettant une insertion aisée dans le canal opérateur de l'endoscope, qui est de quelques millimètres de diamètre suivant l'application clinique (entre 2 mm et 4 mm). On peut utiliser un faisceau constitué d'une fibre optique souple unique ou bien d'une pluralité de fibres. En pratique, le diamètre utile peut être choisi en fonction de la tache de focalisation de la tête optique, par exemple pour qu'elle soit de l'ordre de plusieurs centaines de microns dans lesquels on sait que la nature du tissu biologique observé ne diffère pas d'une cellule à une autre.

En sortie du faisceau de fibres 3, le signal d'excitation traverse la tête optique 4. Celle-ci comprend plusieurs moyens optiques, permettant de faire converger le signal d'excitation, et deux lames de verre (non représentés sur les figures), l'une en commun avec la sortie du faisceau de fibres 3 et l'autre en contact avec le tissu afin de réaliser une adaptation d'indice avec les tissus biologiques.

Les moyens optiques présentent les caractéristiques suivantes :
- permettre une analyse du tissu à une profondeur de plusieurs dizaines à plusieurs centaines de microns ;
- minimisation des aberrations afin de transcrire la PSF en sortie du faisceau de fibres sur le tissu sans élargir celle-ci ou la déformer ;
- optimisation du taux de couplage en retour dans le faisceau de fibres en optimisant la qualité du front d'onde ;
- minimisation de la réflexion parasite s'opérant en sortie du faisceau de fibres par l'intégration d'une lame de verre.

De plus, s'il s'agit d'une tête optique destinée à un endoscope, ses dimensions doivent être compatibles avec celle du canal opérateur de l'endoscope.

Le bloc optique de focalisation est constitué d'un système de lentilles à grandissement unitaire ou non, formant un objectif sur-mesure ou un système comprenant par exemple deux objectifs de microscopes.

Selon l'invention, les fibres du faisceau 3 ont pour fonction également de détecter le signal émis par la zone 5 subsurfacique. En sortie du faisceau de fibres 3, le signal détecté est, comme on l'a vu réfléchi par la lame dichroïque D et dirigé vers la fente 21 d'un spectrographe 20. Le couplage du signal de fluorescence à la fente 21 du spectrographe est réalisé grâce à un doublet achromatique. En variante, on peut utiliser toute autre optique achromatique, l'analyse du signal de fluorescence s'effectuant sur un large domaine spectral (350nm-650nm). Un filtre passe-haut 22 permet ainsi de supprimer la longueur d'onde d'excitation (la lumière rétrodiffusée par le tissu à la même longueur d'onde que la longueur d'onde d'excitation est en effet beaucoup plus importante que la lumière d'autofluorescence des tissus qui se produit à des longueurs d'onde plus grandes). Par conséquent, afin de ne pas saturer le détecteur, on bloque la lumière rétrodiffusée par le filtre passe-haut et une lentille L2, placée en amont du filtre passe-haut 22, permet d'améliorer le rapport signal à bruit en augmentant le signal détecté par l'adaptation du faisceau de retour aux dimensions de la fente 21.

Le spectrographe 20 est choisi de manière à présenter une grande ouverture numérique, un refroidissement par effet Peltier, un faible bruit afin d'augmenter le rapport signal à bruit, ainsi qu'une bonne résolution spectrale (de l'ordre de 3 nm). Des moyens de visualisation de spectre et d'analyse et de traitement sont en outre prévus.

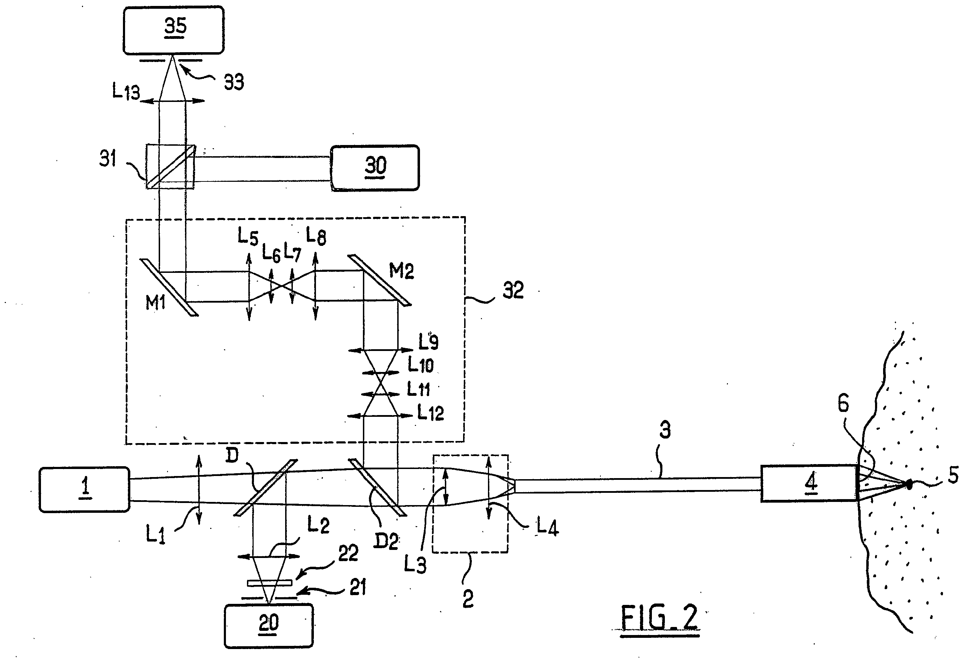

A la figure 2, est représenté schématiquement l'appareillage de la figure 1 auquel on a couplé avantageusement un système d'imagerie confocale. L'appareillage comprend donc une voie spectroscopique qui correspond à celle décrite en référence à la figure 1 (les mêmes références sont utilisées) et une voie supplémentaire tomographique permettant de réaliser conjointement une image confocale du site d'analyse. Les deux voies utilisent avantageusement en commun le faisceau de fibres optiques 3 et la tête optique 4, ainsi que le moyen d'injection 2 dans ledit faisceau de fibres 3.

De manière spécifique, pour obtenir une image confocale point par point, l'appareillage utilise ici un faisceau 3 comprenant plusieurs fibres optiques qui sont illuminées une à une et tour à tour de manière adressée. On peut utiliser tout faisceau présentant suffisamment de fibres et un faible espacement inter-coeur afin d'obtenir une bonne résolution spatiale. A titre d'exemple, on peut utiliser un toron de fibres optiques de marque Sumitomo^{®} constitué de 30 000 fibres de diamètre de coeur de 2,5 *µ*m et d'espacement inter-coeur de 4 *µ*m, ou bien un toron de marque Fujikura^{®} constitué de 30 000 fibres de diamètre de coeur de 2*µ*m et d'espacement inter-coeur de 3,7 *µ*m. De tels faisceaux de fibres sont compatibles avec une application endoscopique. Pour la voie spectroscopique, l'acquisition du spectre avec de tels faisceaux de fibres se fera sur la totalité des fibres du faisceau qui correspond à une zone d'analyse de plusieurs centaines de microns dans laquelle la nature du tissu ne diffère pas d'une cellule à une autre.

Egalement de manière spécifique, la tête optique est adaptée à une imagerie confocale fibre à fibre permettant d'obtenir une zone d'analyse 5 focalisée de l'ordre de 0,5 mm de diamètre dans un plan d'analyse en coupe XY.

En amont du faisceau de fibres 3, la voie tomographique comporte une source 30 constituée d'une diode laser à 683 nm et présentant une très bonne qualité de front d'onde. Cette diode est pulsée afin de dissocier par détection synchrone le signal utile de la réflexion parasite qui s'opère à l'entrée du faisceau de fibres 3. On pourrait aussi utiliser un laser solide ou à gaz, mais le choix en longueur d'onde dans la bande 600-800 nm où l'absorption dans les tissus est moindre, est moins étendu ; de plus, le coût à puissance équivalente est bien plus important.

Pour séparer le signal d'illumination et le signal retour d'analyse, on utilise un moyen de séparation constitué ici d'un cube séparateur 31 50/50 pour des commodités de réglage. On peut aussi utiliser une lame séparatrice 50/50.

L'appareillage comporte un système de balayage 32 dont le but est de reproduire une matrice de diodes de même qualité optique que la diode laser de la source et que l'on injectera fibre à fibre. Ceci nécessite une combinaison de moyens optiques non standards permettant de corriger les aberrations présentes dans le système de transport et de duplication de source afin d'éclairer le faisceau 3 fibre par fibre. Cette technique d'imagerie point à point (chaque point correspondant à l'illumination d'une fibre) permet d'obtenir une image confocale de très bonne qualité et à une cadence d'image appropriée (15 images/s).

Le système de balayage est constitué de deux miroirs M1 et M2, l'un est un miroir résonant à une fréquence de 4 kHz ou 8 kHz, l'autre un miroir galvanométrique avec une fréquence variable entre 0 et 300 Hz, et de deux systèmes optiques chacun constitué de quatre lentilles, respectivement L5, L6, L7 et L8, et L9, L10, L11 et 12 permettant de conjuguer les deux miroirs dans un premier temps, puis le miroir M2 et l'entrée du faisceau de fibres 3. Ces systèmes optiques ne doivent pas présenter d'aberrations qui pourraient :
- élargir la PSF après le système d'injection et ainsi dégrader le couplage dans le faisceau de fibres 3 ;
- faire propager du flux dans la gaine qui dégraderait la PSF en bout du faisceau de fibres 3 et de ce fait la résolution de l'appareillage.

A la différence de l'appareillage de la figure 1, le moyen d'injection 2 dans le faisceau de fibres 3 doit présenter ici une PSF égale au diamètre de coeur d'une fibre afin de pouvoir réaliser un couplage optimal avec une seule fibre.

Les lentilles L6-L7 et L10-L11 du système de balayage 32 sont deux doublets correcteurs identiques placés symétriquement par rapport au plan image. Ils permettent, avec le doublet L3 du système d'injection 2 d'obtenir une image de très bonne qualité en éliminant les aberrations résiduelles de la combinaison optique L5 , L8, L9, L12 et L4, et d'uniformiser le taux de couplage dans le faisceau de fibres 3 en supprimant la courbure de champ. Elles permettent également de ce fait d'améliorer la résolution spatiale de l'appareillage en formant une tache dont la PSF est égale au diamètre de coeur des fibres, ce qui n'entraîne pas de propagation de lumière dans la gaine du faisceau de fibres 3 et donc une PSF en sortie dudit faisceau 3 identique à celle d'entrée.

L'appareillage comporte un système de filtrage spatial constitué d'une lentille L13 et d'un trou de filtrage 33 permettant de ne sélectionner que la fibre d'illumination et non les fibres adjacentes qui peuvent générer un signal parasite. La taille du trou de filtrage est telle qu'elle correspond au diamètre de coeur d'une fibre au grandissement près du système optique entre l'entrée du faisceau de fibres 3 et le trou de filtrage 33.

Le faisceau de fibres 3 est équipé à ces deux extrémités d'une lame de verre suffisamment épaisse et d'indice proche de celui des fibres afin de rejeter les réflexions parasites en dehors du trou de filtrage placé devant le détecteur pour la réflexion qui s'opère à l'entrée du faisceau de fibres 3, et en dehors des fibres optiques illuminées pour la réflexion qui s'opère en sortie du faisceau de fibres 3. Les lames de verre sont traitées anti-reflet afin de minimiser la lumière réfléchie.

Comme détecteur de signal 35 on utilise une photodiode à avalanche qui acquiert le signal en continu, ce qui nécessite de ramener le signal parasite provenant des deux extrémités du faisceau de fibres 3 au même ordre de grandeur que le signal utile afin de ne pas saturer le détecteur. La suppression du résidu de réflexion parasite à l'entrée du faisceau de fibres 3 est alors effectuée par un filtrage temporel numérique. Tout autre photodétecteur monopixel tel que le photomultiplicateur peut être utilisé, l'avantage de la photodiode à avalanche étant son rendement quantique de détection plus élevé que celui des autres détecteurs.

Afin de procéder au couplage des deux voies imagerie confocale/spectroscopie qui s'effectue avec deux sources de longueurs d'onde différentes, une lame dichroïque D2 réfléchissant le rouge et transmettant le bleu et le vert est utilisée. Le couplage pourrait aussi être réalisé en transmission dans le rouge et en réflexion dans le bleu et le vert, mais il présente des taux de réflexion et transmission moins optimales dans ce sens.

En fonctionnement, l'acquisition d'un spectre peut se faire simultanément à l'acquisition d'une image tomographique. L'appareillage comporte des moyens d'analyse et de traitement qui permettent à partir des signaux détectés par le détecteur de signal 35 de recréer une image numérique.

La résolution spatiale que l'on peut obtenir est de l'ordre de 5*µ*m. Elle permet notamment le diagnostic de lésions pré-cancéreuses basé sur la taille, la forme et la densité des noyaux observés.

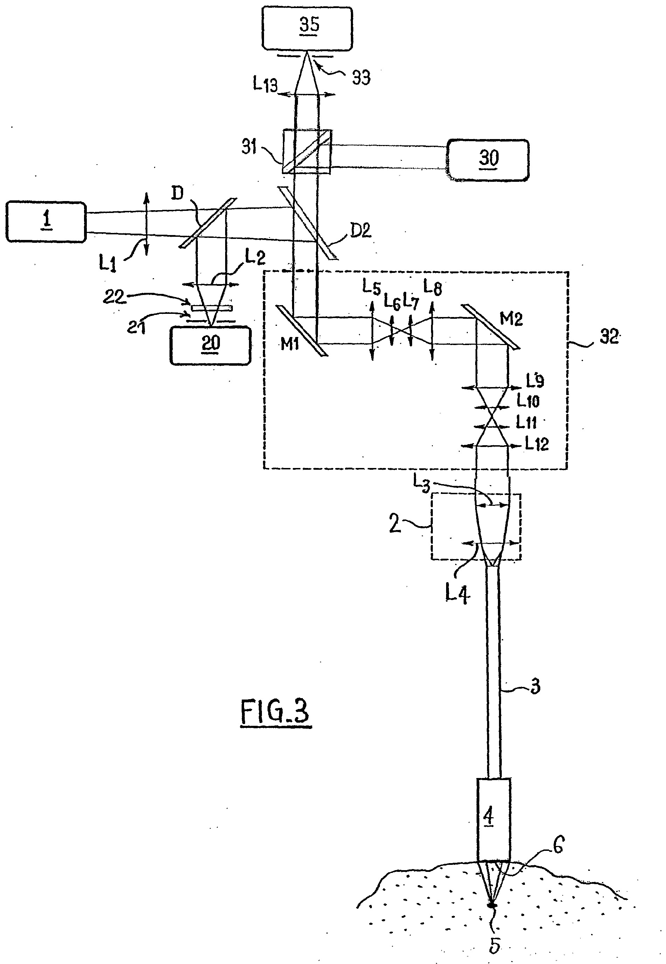

Sur la figure 3, est représentée une variante de réalisation de l'appareillage de la figure 2. Les éléments identiques portent les mêmes références sur les deux figures. Selon cette variante, le couplage des deux voies imagerie confocale / spectroscopie est réalisé en amont des moyens de balayage 32. A cet effet, la lame dichroïque D2 est placée en amont du miroir M1. L'avantage de cette construction est quelle permet d'utiliser les moyens de balayage 32 pour déplacer un faisceau d'excitation émis sur la voie spectroscopique possédant un diamètre utile plus petit que le diamètre total du faisceau de fibres 3, de manière à l'injecter en une position différente sur la face d'entrée du faisceau 3. Cela permet par exemple de déplacer la zone d'analyse spectroscopique pour notamment la faire correspondre à une zone d'image obtenue par la voie de l'imagerie confocale.

En variante encore, pouvant s'appliquer aux appareillages des figures 1 à 3, on peut prévoir en remplacement de la lentille d'adaptation L1 un système afocal permettant de modifier la taille du faisceau d'excitation pour le faire correspondre à un sous-groupe donné de fibres optiques du faisceau 3.

## Revendications

1. Appareillage d'analyse spectroscopique d'autofluorescence d'un tissu biologique comprenant :
- une source d'excitation (1) émettant un faisceau d'excitation constituant un signal d'excitation,
- un faisceau (3) constitué d'une pluralité de fibres optiques souples,
- un moyen d'injection (2) du signal d'excitation produit par ladite source d'excitation (1) dans ledit faisceau (3) de fibres optiques souples,
- en sortie du faisceau (3) de fibres optiques souples une tête optique (4) destinée à être placée au contact du tissu biologique (6), ladite tête optique (4) étant munie de moyens optiques adaptés à faire converger le signal d'excitation sortant dudit faisceau (3) de fibres optiques souples en une zone d'analyse subsurfacique (5), les mêmes fibres optiques ayant servi à véhiculer le signal d'excitation étant utilisées pour détecter le signal d'autofluorescence émis par ladite zone d'analyse subsurfacique (5),
- des moyens (D) placés en amont du moyen d'injection (2) prévus pour séparer la longueur d'onde du signal d'excitation et la longueur d'onde du signal d'autofluorescence, et
- un moyen d'analyse (21, 22) du signal d'autofluorescence émis,
**caractérisé en ce qu'**il comprend :
des moyens pour régler le diamètre du faisceau d'excitation émis par la source d'excitation (1) et pour exciter en continu soit l'ensemble des fibres optiques soit un sous-groupe de fibres optiques permettant ainsi d'obtenir une taille appropriée pour la zone d'excitation formée sur le tissu.
- des movens (D) placés en amont du moven d'iniection (2)

2. Appareillage selon la revendication 1, **caractérisé en ce que** les moyens optiques de la tête optique (4) comprennent un système de lentilles formant un objectif de focalisation adapté à transcrire la répartition spatiale de la tache focale (PSF) en sortie du faisceau de fibres et la qualité du front d'onde (WFE) et à minimiser la réflexion parasite s'opérant en sortie du faisceau de fibres.

3. Appareillage selon la revendication 1 ou 2, **caractérisé en ce que** la tête optique (4) comprend une lame de verre destinée à venir en contact du tissu biologique à analyser et adaptée à réaliser une adaptation d'indice avec ledit tissu.

4. Appareillage selon l'une quelconque des revendications précédentes, **caractérisé en ce qu'**il comporte une lame de verre placée en sortie du faisceau (3) de fibres optiques et commune avec la tête optique (4), ladite lame étant suffisamment épaisse pour rejeter les réflexions parallèles parasites en sortie dudit faisceau de fibres (3).

5. Appareillage selon l'une quelconque des revendications précédentes, **caractérisé en ce que** le moyen d'injection (2) dans le faisceau (3) de fibres optiques présente une qualité de front d'onde et une répartition spatiale de l'intensité de la tache focale adaptée au diamètre utile du faisceau de fibres (3).

6. Appareillage selon l'une quelconque des revendications précédentes, **caractérisé en ce que** la source d'excitation (1) émet à une longueur d'onde adaptée à exciter des fluorophores endogènes choisis présents dans les tissus biologiques du site observé.

7. Appareillage selon l'une quelconque des revendications précédentes, **caractérisé en ce que** le moyen pour séparer les longueurs d'onde est une lame dichroïque (D).

8. Appareillage selon l'une quelconque des revendications précédentes, **caractérisé en ce que** le moyen d'analyse spectroscopique comprend un spectrographe (20) et un moyen de couplage (21) à la fente du spectrographe.

9. Appareillage selon la revendication 8, **caractérisé en ce que** le moyen de couplage (21) à la fente du spectrographe comprend un moyen optique achromatique.

10. Appareillage selon la revendication 8 ou 9, **caractérisé par** un moyen de réjection (22) placé en amont du moyen de couplage (21) et adapté à supprimer la longueur d'onde d'excitation rétroémise.

11. Appareillage selon la revendication 10, **caractérisé par** une lentille (L2) placée en amont du moyen de réjection (22) adaptée à améliorer le rapport signal à bruit.

12. Appareillage selon l'une quelconque des revendications précédentes, **caractérisé en ce qu'**il comporte un moyen d'adaptation (L1) de la taille du faisceau émis par la source d'excitation (1) au diamètre utile du faisceau de fibres optiques (3).

13. Appareillage selon l'une quelconque des revendications précédentes, **caractérisé en ce qu'**il comporte en outre des moyens pour réaliser conjointement une image confocale de la zone d'analyse (5), comprenant :
- une source d'illumination (30),
- un détecteur (35) du signal de retour pour analyse,
- un moyen de séparation (31) du signal d'illumination et dudit signal de retour,
- des moyens pour coupler (D2) le faisceau d'excitation pour l'analyse spectroscopique et le faisceau d'illumination pour l'imagerie confocale, avant l'entrée dans le moyen d'injection (2) dans le faisceau de fibres optiques (3),
- un moyen (32) de balayage rapide des fibres une à une situé en amont du moyen d'injection dans le faisceau de fibres (3), et
- un système de filtrage (33) spatial à l'entrée du détecteur (35) de signal adapté à sélectionner le signal de retour provenant de la fibre illuminée,
le moyen d'injection (2) dans le faisceau de fibres (3) présentant une répartition spatiale de l'intensité de la tache focale égale au diamètre de coeur d'une fibre, chaque fibre étant illuminée tour à tour et de manière adressée.

14. Appareillage selon la revendication 13, **caractérisé en ce que** les moyens de couplage sont placés en amont du moyen de balayage (32).

15. Appareillage selon la revendication 13 ou 14, **caractérisé en ce que** la source d'illumination (30) est une diode laser pulsée.

16. Appareillage selon l'une des revendications 13 à 15, **caractérisé en ce que** la source d'illumination présente une qualité de front d'onde de l'ordre de λ/8.

17. Appareillage selon l'une des revendications 13 à 16, **caractérisé en ce que** le détecteur (35) du signal de retour est une photodiode à avalanche.

18. Appareillage selon l'une quelconque des revendications 13 à 17, **caractérisé en ce que** les moyens de couplage (31) du signal d'excitation pour l'analyse spectroscopique et du signal d'illumination pour l'imagerie confocale, comprennent une lame dichroïque (D2).

19. Appareillage selon l'une quelconque des revendications 13 à 18, **caractérisé en ce que** le moyen (32) de balayage rapide des fibres une à une comprend un miroir résonant (M1) à une fréquence donnée et un miroir galvanométrique (M2) avec une fréquence variable, et deux systèmes optiques chacun constitué de lentilles (L5-8, L9-12) adaptées à conjuguer les deux miroirs (M1, M2) dans un premier temps puis le miroir galvanométrique (M2) et l'entrée du faisceau de fibres (3) dans un deuxième temps.

20. Appareillage selon l'une quelconque des revendications 13 à 19, **caractérisé en ce que** le système de filtrage spatial comprend un trou de filtrage (33) dont la taille est telle qu'elle correspond au diamètre de coeur d'une fibre au grandissement près du système optique entre l'entrée du faisceau de fibres (3) et le trou de filtrage (33).

## Patentansprüche

1. Analysevorrichtung zur Autofluoreszenzspektroskopie eines biologischen Gewebes, enthaltend:
- eine Anregungsquelle (1), die ein ein Anregungssignal bildendes Anregungsstrahlenbündel ausgibt,
- ein Bündel (3), das aus einer Mehrzahl von nachgiebigen Lichtleitfasern besteht,
- eine Einspeiseeinrichtung (2) zum Einspeisen des von der Anregungsquelle (1) erzeugten Anregungssignals in das Bündel (3) nachgiebiger Lichtleitfasern,
- am Austritt des Bündels (3) aus nachgiebigen Lichtleiterfasern einen optischen Kopf (4), der dazu bestimmt ist, in Kontakt mit dem biologischen Gewebe (6) platziert zu werden, wobei der optische Kopf (4) mit optischen Einrichtungen versehen ist, die dazu ausgelegt sind, das aus dem Bündel (3) nachgiebiger Lichtleiterfasern austretende Anregungssignal an einem unter der Oberfläche liegenden Analysebereich (5) konvergieren zu lassen, wobei die gleichen Lichtleitfasern, die dazu gedient haben, das Anregungssignal weiterzuleiten, dazu verwendet werden, das Autofluoreszenzsignal zu erfassen, das von dem unter der Oberfläche liegenden Analysebereich (5) ausgegeben wurde,
- Einrichtungen (D), die der Einspeiseeinrichtung (2) vorgelagert und dazu vorgesehen sind, die Wellenlänge des Anregungssignals und die Wellenlänge des Autofluoreszenzsignals zu trennen, und
- eine Analyseeinrichtung (21, 22) zur Analyse des ausgegebenen Autofluoreszenzsignals,
**dadurch gekennzeichnet, dass** sie enthält:
Einrichtungen zum Einstellen des Durchmessers des von der Anregungsquelle (1) ausgegebenen Anregungsbündels und zum kontinuierlichen Anregen entweder der gesamten Lichtleitfasern oder einer Untergruppe von Lichtleitfasern, sodass eine geeignete Größe für den am Gewebe gebildeten Anregungsbereich erhalten werden kann.

2. Vorrichtung nach Anspruch 1, **dadurch gekennzeichnet, dass** die optischen Einrichtungen des optischen Kopfes (4) ein Linsensystem enthalten, das ein Fokussierungsobjektiv bildet, das dazu ausgelegt ist, die räumliche Verteilung des Brennflecks (PSF) am Austritt des Lichtleiterfaserbündels und die Güte der Wellenfront (WFE) zu übertragen und die Störreflexion zu minimieren, die am Austritt des Faserbündels erfolgt.

3. Vorrichtung nach Anspruch 1 oder 2, **dadurch gekennzeichnet, dass** der optische Kopf (4) eine Glasplatte enthält, die dazu bestimmt ist, mit dem zu analysierenden biologischen Gewebe in Kontakt zu gelangen, und die dazu ausgelegt ist, eine Indexanpassung mit dem Gewebe durchzuführen.

4. Vorrichtung nach einem der vorangehenden Ansprüche, **dadurch gekennzeichnet, dass** sie eine Glasplatte enthält, die gemeinsam am Austritt des Lichtleiterfaserbündels (3) mit dem optischen Kopf (4) angeordnet ist, wobei die Platte dick genug ist, um die parallel verlaufenden Störreflexionen am Austritt des Faserbündels (3) zu unterdrücken.

5. Vorrichtung nach einem der vorangehenden Ansprüche, **dadurch gekennzeichnet, dass** die Einspeiseeinrichtung (2) zum Einspeisen in das Lichtleiterfaserbündel (3) eine Wellenfrontgüte und eine räumliche Verteilung der Stärke des Brennflecks aufweist, die auf den Nutzdurchmesser des Faserbündels (3) angepasst ist.

6. Vorrichtung nach einem der vorangehenden Ansprüche, **dadurch gekennzeichnet, dass** die Anregungsquelle (1) auf einer Wellenlänge emittiert, die dazu ausgelegt ist, in den biologischen Geweben der Beobachtungsstelle vorhandene, ausgewählte endogene Fluorophore anzuregen.

7. Vorrichtung nach einem der vorangehenden Ansprüche, **dadurch gekennzeichnet, dass** die Einrichtung zum Trennen der Wellenlängen eine dichroitische Platte (D) ist.

8. Vorrichtung nach einem der vorangehenden Ansprüche, **dadurch gekennzeichnet, dass** die Spektroskopieanalyseeinrichtung einen Spektrographen (20) und eine Kopplungseinrichtung (21) zum Koppeln mit dem Spalt des Spektrographen enthält.

9. Vorrichtung nach Anspruch 8, **dadurch gekennzeichnet, dass** die Kopplungseinrichtung (21) zum Koppeln mit dem Spalt des Spektrographen eine achromatische optische Einrichtung enthält.

10. Vorrichtung nach Anspruch 8 oder 9, **gekennzeichnet durch** eine Sperreinrichtung (22), die der Koppeleinrichtung (21) vorgelagert und dazu ausgelegt ist, die Wellenlänge der Rückstrahlung zu unterdrücken.

11. Vorrichtung nach Anspruch 10, **gekennzeichnet durch** eine Linse (L2), die der Sperreinrichtung (22) vorgelagert und dazu ausgelegt ist, das Signal-zu-Rausch-Verhältnis zu verbessern.

12. Vorrichtung nach einem der vorangehenden Ansprüche, **dadurch gekennzeichnet, dass** sie eine Anpassungseinrichtung (L1) zum Anpassen der Größe des von der Anregungsquelle (1) emittierten Bündels auf den Nutzdurchmesser des Lichtleitfaserbündels (3) enthält.

13. Vorrichtung nach einem der vorangehenden Ansprüche, **dadurch gekennzeichnet, dass** sie ferner Einrichtungen enthält, um gemeinsam ein konfokales Bild des Analysebereichs (5) zu erstellen, die enthält:
- eine Beleuchtungsquelle (30),
- einen Sensor (35) für das Rücksignal zur Analyse,
- eine Trenneinrichtung (31) zum Tennen des Beleuchtungssignals vom Rücksignal,
- Kopplungsmittel (D2) zum Koppeln des Anregungsbündels zur spektroskopischen Analyse und des Beleuchtungsbündels zur konfokalen Abbildung vor dem Eintritt in die Einspeiseeinrichtung (2) zum Einspeisen in das Lichtleitfaserbündel (3),
- eine Schnellabtasteinrichtung (32) zum schnellen sukzessiven Abtasten der Fasern, die der Einspeiseeinrichtung zum Einspeisen in das Faserbündel (3) vorgelagert ist, und
- ein Filtersystem (33) zur räumlichen Filterung am Eingang des Signalsensors (35), das dazu ausgelegt ist, das Rücksignal auszuwählen, das von der beleuchteten Faser stammt,
wobei die Einspeiseeinrichtung (2) zum Einspeisen in das Faserbündel (3) eine räumliche Verteilung der Stärke des Brennflecks aufweist, die gleich dem Kerndurchmesser einer Faser ist, wobei jede Faser der Reihe nach und in gezielter Weise beleuchtet wird.

14. Vorrichtung nach Anspruch 13, **dadurch gekennzeichnet, dass** die Kopplungseinrichtungen der Abtasteinrichtung (32) vorgelagert sind.

15. Vorrichtung nach Anspruch 13 oder 14, **dadurch gekennzeichnet, dass** die Beleuchtungsquelle (30) eine gepulste Laserdiode ist.

16. Vorrichtung nach einem der Ansprüche 13 bis 15, **dadurch gekennzeichnet, dass** die Beleuchtungsquelle eine Wellenfrontgüte in der Größenordnung von λ/8 aufweist.

17. Vorrichtung nach einem der Ansprüche 13 bis 16, **dadurch gekennzeichnet, dass** der Sensor (35) für das Rücksignal eine Lawinen-Photodiode ist.

18. Vorrichtung nach einem der Ansprüche 13 bis 17, **dadurch gekennzeichnet, dass** die Kopplungseinrichtung (31) zum Einkoppeln des Anregungssignals zur spektroskopischen Analyse und des Beleuchtungssignals zur konfokalen Abbildung eine dichroitische Platte (D2) enthält.

19. Vorrichtung nach einem der Ansprüche 13 bis 18, **dadurch gekennzeichnet, dass** die Schnellabtasteinrichtung (32) zum schnellen Abtasten der Fasern eine nach der anderen einen Resonatorspiegel (M1) mit gegebener Frequenz und einen galvanometrischen Spiegel (M2) mit variabler Frequenz enthält, sowie zwei optische Systeme, die jeweils aus Linsen (L5-8, L9-12) bestehen, die dazu ausgebildet sind, zunächst die beiden Spiegel (M1, M2) und dann den galvanometrischen Spiegel (M2) und den Eintritt des Faserbündels (3) zu verknüpfen.

20. Vorrichtung nach einem der Ansprüche 13 bis 19, **dadurch gekennzeichnet, dass** das System zur räumlichen Filterung eine Filteröffnung (33) aufweist, deren Größe derart ist, dass sie dem Kerndurchmesser einer Faser auf die Vergrößerung genau des optischen Systems zwischen dem Eintritt des Faserbündels (3) und der Filteröffnung (33) entspricht.

## Claims

1. Equipment for spectroscopic analysis of autofluorescence of a biological tissue comprising:
- an excitation source (1) emitting an excitation beam constituting an excitation signal,
a bundle (3) constituted by a plurality of flexible optical fibres,
- means for injecting (2) the excitation signal produced by said excitation source (1) into said bundle of flexible optical fibres,
- at output of said bundle (3) of flexible optical fibres an optical head (4) intended to be placed in contact with the biological tissue (6), said optical head being equipped with optical means adapted for converging the excitation signal coming out of said bundle (3) of flexible optical fibres into a subsurface analysis zone (5), the same optical fibres having served for carrying the excitation signal being used for detecting the autofluorescence signal emitted by said subsurface analysis zone (5),
- means (D) placed upstream of the means for injecting (2) being moreover provided for separating the excitation signal wavelength and the autofluorescence signal wavelength, and
- a means for analyzing (21, 22) an emitted autofluorescence signal,
**characterized in that** it comprises:
means for adjusting the diameter of the excitation beam emitted by the excitation source (1) and for continuously exciting either all of the fibres or a sub-group of fibres making it possible to obtain an appropriate size for the excitation zone formed on the tissue.

2. Equipment according to claim 1, **characterized in that** the optical means of the optical head (4) comprise a system of lenses forming a focussing objective adapted for transcribing the spatial distribution of the focal spot (PSF) at the fibre bundle output and the quality of the wave front (WFE) and for minimizing the parasitic reflection occurring at the fibre bundle output.

3. Equipment according to claim 1 or 2, **characterized in that** the optical head (4) comprises a glass plate intended to come into contact with the biological tissue to be analyzed and adapted for producing an index adaptation with said tissue.

4. Equipment according to any one of the preceding claims, **characterized in that** it comprises a glass plate placed at the output of the optical fibre bundle (3) and shared with the optical head (4), said plate being sufficiently thick to reject the parasitic parallel reflections at the output of said fibre bundle (3).

5. Equipment according to any one of the preceding claims, **characterized in that** the means for injecting (2) into the optical fibre bundle (3) has a wave front quality and a spatial distribution of the focal spot intensity adapted to the useful diameter of the fibre bundle (3).

6. Equipment according to any one of the preceding claims, **characterized in that** the excitation source (1) emits at a wavelength adapted to excite chosen endogenous fluorophores present in the biological tissues of the observed site.

7. Equipment according to any one of the preceding claims, **characterized in that** the means for separating the wavelengths is a dichroic plate (D).

8. Equipment according to any one of the preceding claims, **characterized in that** the means for spectroscopic analysis comprise a spectrograph (20) and a means of coupling (21) to the slit of the spectrograph.

9. Equipment according to claim 8, **characterized in that** the means for coupling (21) to the slit of the spectrograph comprises an achromatic optical

10. Equipment according to claim 8 or 9, **characterized by** a means for rejecting (22) placed upstream of the coupling means (21) and adapted for eliminating the backscattered excitation wavelength.

11. Equipment according to claim 10, **characterized by** a lens (L2) placed upstream of the means for rejecting (22) adapted for improving the signal-to-noise ratio.

12. Equipment according to any one of the preceding claims, **characterized in that** it comprises a means for adapting (L1) the size of the beam emitted by the excitation source (1) to the useful diameter of the optical fibre bundle (3).

13. Equipment according to any one of the preceding claims, **characterized in that** it moreover comprises means for jointly producing a confocal image of the analysis zone (5), comprising:
- an illumination source (30),
- a detector (35) of the return signal for analysis,
- a means for separating (31) the illumination signal and said return signal,
- means for coupling (D2) the excitation beam for the spectroscopic analysis and the illumination beam for the confocal imaging, before introduction into the means for injecting (2) into the optical fibre bundle (3),
- a means (32) for rapid scanning one by one of the fibres situated upstream of the means for injecting into the fibre bundle (3), and
- a system for spatial filtering (33) at the input to the signal detector (35) adapted for selecting the return signal originating from the fibre illuminated,
the means for injecting (2) into the fibre bundle (3) having a spatial distribution of the focal spot intensity equal to the diameter of a fibre core, each fibre being illuminated alternately and in an addressed manner.

14. Equipment according to claim 13, **characterized in that** the means for coupling are placed upstream of the scanning means (32).

15. Equipment according to claim 13 or 14, **characterized in that** the illumination source (30) is a pulsed laser diode.

16. Equipment according to one of claims 13 to 15, **characterized in that** the illumination source has a wave front quality of the order of λ/8.

17. Equipment according to one of claims 13 to 16, **characterized in that** the detector (35) of the return signal is an avalanche photodiode.

18. Equipment according to any one of claims 13 to 17, **characterized in that** the means for coupling (31) the excitation signal for the spectroscopic analysis and the illumination signal for the confocal imaging, comprise a dichroic plate (D2).

19. Equipment according to any one of claims 13 to 18, **characterized in that** the means (32) for rapid scanning of the fibres one by one comprises a mirror (M1) resonating at a given frequency and a galvanometric mirror (M2) with a variable frequency, and two optical systems each constituted by lenses (L5-8,L9-12) first adapted for conjugating the two mirrors (M1,M2) then the galvanometric mirror (M2) and the fibre bundle (3) input.

20. Equipment according to any one of claims 13 to 19, **characterized in that** the spatial filtering system comprises a filtering hole (33) the size of which is such that it corresponds to the diameter of a fibre core, taking into account the magnification of the optical system, between the fibre bundle (3) input and the filtering hole (33).
